# EUROPEAN PATENT APPLICATION

(11) **EP 4 585 700 A2**
(43) Date of publication of application: **16.07.2025**
(21) Application number: 24223070.4
(22) Date of filing: 23.12.2024
(51) Int. Cl.: C12Q 1/6853

(54) **ISOTHERMAL NUCLEIC ACID AMPLIFICATION USING LNA MODIFIED PRIMERS**

(30) Priority: 23.12.2023 PL 44727923
(71) Applicant: Instytut Biotechnologii I Medycyny Molekularnej, 80-180 Gdansk (PL); Geneme Molecular Sp. z o.o., 80-180 Gdansk (PL)
(72) Inventor: NIDZWORSKI, Dawid, 80-180 Gdansk (PL); ZOLEDOWSKA, Sabina, 80-180 Gdansk (PL); SZEMIAKO, Kasjan, 80-287 Gdansk (PL); DOMORADZKI, Tomasz, 02-775 Warszawa (PL); SKWARECKA, Marta, 80-180 Gdansk (PL)
(74) Representative: Matyka, Malgorzata

(57) **Abstract**

The subject of the present invention is a new conformation of LNA-type modifications in primers used in loop-mediated isothermal amplification (LAMP) that allows specific identification of single-nucleotide mutations (SNPs) in genomic DNA, and a method for its use.

## Description

The subject of the present invention is a new conformation of LNA-type modifications in oligonucleotides that allows specific identification of single-nucleotide mutations (SNPs) in genomic DNA, and a method for its use.

Single Nucleotide Polymorphism (SNP) is one of the key sources of genetic variation. This phenomenon involves the modification of a single nucleotide, leading to variations in the sequence of the same gene between two individuals of the same species. On average, 4 to 5 million such variations can be found in the genome, meaning that they occur on average once every thousand base pairs. Most such variations do not affect the health or development of the organism. However, when a variation localizes in the coding or regulatory region of a gene, it can affect its function and be associated with the development of diseases. That's why it's important to create systems that can quickly and specifically identify single-nucleotide modifications in genomic DNA.

One approach in improving the specificity and simplifying the method for identifying polymorphisms in DNA is the use of isothermal amplification reaction. Isothermal amplification (including Loop-Mediated Isothermal Amplification of DNA (LAMP)) is an effective amplification method, providing a quick and simple diagnostic tool. It is based on a set of several pairs of specially designed primers and a DNA polymerase with strand substitution activity. Amplification products contain single-stranded loops with repeated target sequences, which can be detected by electrophoretic, densitometric, colorimetric or real-time intercalating dye-based methods. Detection of specific nucleic acid sequences by the isothermal method eliminates many of the limitations of polymerase chain reaction-PCR-based methods. The LAMP-type method is performed under isothermal conditions, which eliminates the need for thermocyclers and allows to perform the reaction without the need for sophisticated equipment (a heating block or water bath is sufficient). In addition, the simplified method for detecting amplification products makes it suitable for use in the field or in less advanced laboratories. Comparing the LAMP method with other DNA identification methods, mainly PCR, the isothermal method was found to have greater sensitivity and specificity. The LAMP method has some limitations that sometimes discourage researchers from using it, mainly related to the difficulty of designing primers. The selection of primers is a very complex and complicated process, requiring the search of many genomic sequences. This is a key step in the implementation of this method, and only precise selection of primers can determine the efficiency of amplification of a specific sequence. The success rate of systems specifically identifying SNPs can be increased by using modified oligonucleotide pairs, such as LNA, in the reaction.

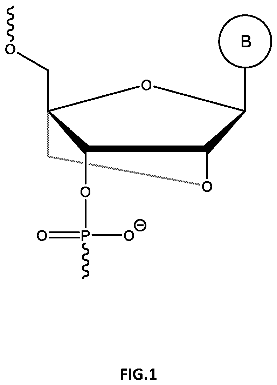

LNA (Locked Nucleic Acid) is a new type of nucleotide, an RNA analog whose synthesis and hybridization was described in 1999. The LNA-type nucleotide contains a methylene bridge that connects the 2'-O atom to the 4'-C atom in the ribose ring (Fig.1) leading to its locking and reducing the conformational flexibility of ribose and changing the local organization of the phosphate backbone. The LNA bases are linked by the same phosphate backbone found in DNA or RNA, allowing LNA oligomers to be obtained using methods as other nucleic acids. LNA's similarity to DNA/RNA synthesis and high solubility allows for the distribution of LNA bases between DNA or RNA bases. LNA oligonucleotides exhibit unprecedented thermal stability when hybridized to a complementary DNA or RNA strand. For each LNA monomer incorporated, the melting point (Tm) of the duplex increases by 2-8°C. This allows LNA oligonucleotides to be shorter than traditional DNA or RNA oligonucleotides and still retain a high Tm. This is important when the oligonucleotide is used to detect small or very similar molecular targets. By creating modified oligonucleotides that combine LNA with DNA or RNA, it is possible to optimize sensitivity and specificity by changing the LNA content of the oligonucleotide. The incorporation of LNA into oligonucleotides has been shown to improve the sensitivity and specificity of many hybridization-based technologies, including PCR, microarrays and in situ hybridization (ISH). Proper placement of LNA monomers and the right amount of these modifications in the primer can provide excellent discrimination of closely related sequences even in the case of a difference of just one nucleotide (SNP). Incorporating LNAs into oligonucleotides can increase the difference between a duplex with a perfect match and a binding with a mismatch by several degrees. The increase in ΔTm enables better discrimination between closely related sequences and thus opens up opportunities for efficient SNP identification.

The subject of the invention is a method of modifying primers for an isothermal amplification reaction using LNA-type nucleotides using the example of the RS1183736 polymorphism in human genomic DNA, in chromosome 1 at position chr1:55717490 (NCBI), which includes:
The nucleotide sequence of the FIP primer shown on SEQ1,
The nucleotide sequence of the BIP_Mut primer identifying the C allele shown on SEQ2,
The nucleotide sequence of the BIP_WT primer identifying the A allele shown on SEQ3,
The nucleotide sequence of the F3 primer shown on SEQ4,
The nucleotide sequence of the B3 primer shown on SEQ5,
The nucleotide sequence of the LoopF primer shown on SEQ6,
The nucleotide sequence of the LoopB primer shown on SEQ7.

A method where the ribose ring in the 5 nucleotides of the primer is blocked: the nucleotide corresponding to the polymorphic allele/mutation and the four nucleotides in the immediate vicinity from the 5' and 3' sides of the oligonucleotide sequence, as shown in SEQ.1 and SEQ.2, by forming a methylene bridge between the 2'-O and 4'-C atoms.

The primers defined above are specific to a polymorphic sequence in which there is a single-nucleotide polymorphism with the C (mutant version) and A (wild-type version) alleles.

Also an object of the invention is a method of detecting and identifying a single-nucleotide mutation, using RS1183736 as an example, in human genomic DNA, comprising the steps:
a) First reaction DNA amplification using primers with SEQ.1, SEQ.2, SEQ.4, SEQ.5, SEQ.6, SEQ.7.
b) The second reaction amplified DNA using primers with SEQ.1, SEQ.3, SEQ.4, SEQ.5, SEQ.6, SEQ.7.
c) Detection of the complexes formed by observing the increase in fluorescence of the intercalating dye, which is added to the reaction mixture.

A method where the target gene material is swab, saliva, blood, tissue or other clinical samples containing genomic DNA.

A kit for use in the manner defined above, including:
a) Starters
b) DNA polymerase with cofactor
c) Deoxyribonucleoside triphosphates
d) Reaction buffer
e) SybrGreen or EvaGreen intercalating dye

A kit containing reagents required for the amplification steps: hybridization and DNA strand synthesis.

Primers containing SEQ.1 - SEQ.7 for use in detecting and identifying a single-nucleotide mutation using RS1183736 as an example in human genomic DNA.

### Description of Figures and Sequences:

**Fig.1** - schematic representation of the LNA-type modification involving "locking" the ribose ring in the nucleotide by forming a methylene bridge between the 2'-O atoms of the 4'-C in the ribose ring.

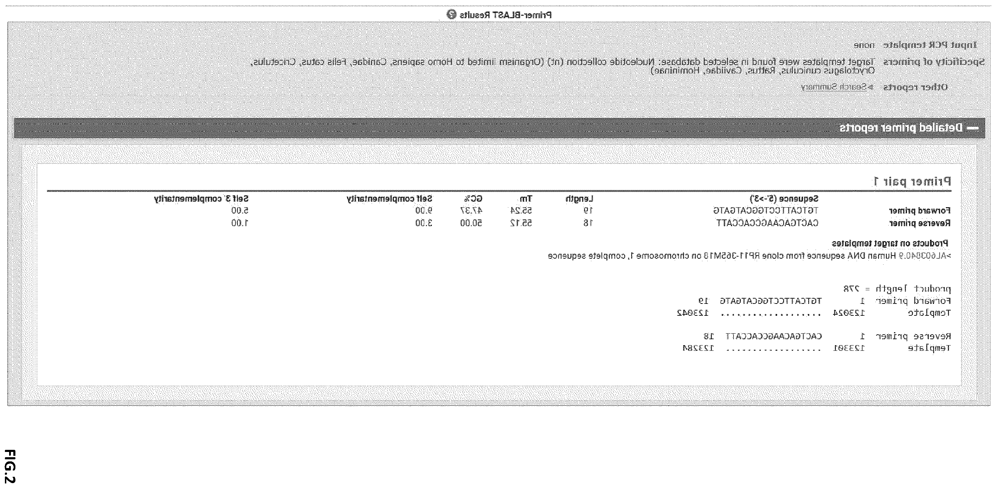

**Fig.2****.** - shows the result of *in silico* specificity analysis of the system identifying the RS1183736 polymorphism against genomic DNA of farmed and domestic animals (canids (Canidae), cats (Felis catus), Hamsters (Cricetulus), Rabbits (Oryctolagus cuniculus), Rats (Rattus), Guinea Pigs (Caviidae), Human Primates (Homininae)) and other human genes than the sequence within chr1:55717490. The study was conducted using NCBI's Primer-Blast program

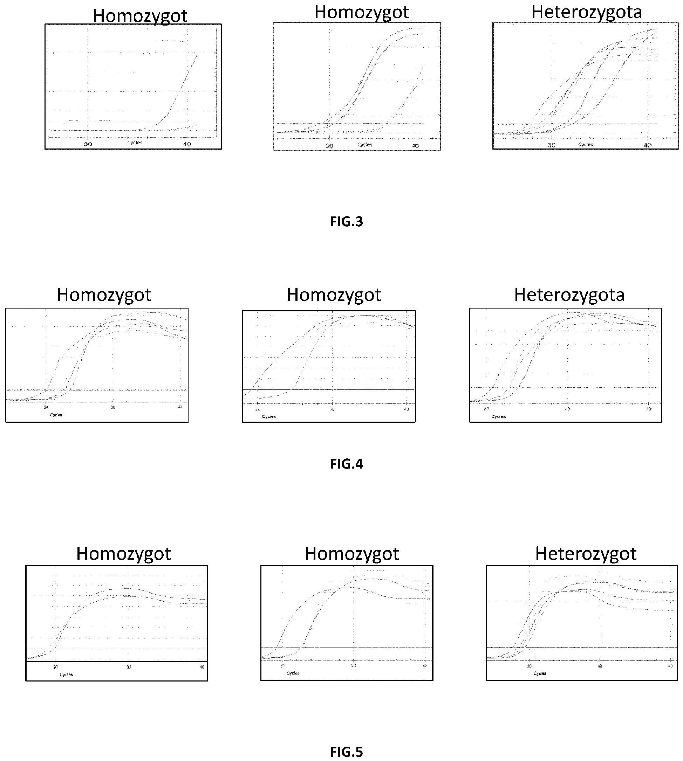

**Fig.3****.** - shows the results of the reaction to identify the RS1183736 polymorphism in human genomic DNA carried out using LNA-type 5-nucleotide modification primers in a real-time isothermal reaction with the addition of SybrGreen intercalating dye. Green indicates the system identifying the A allele, red the C allele.
**Fig.4****.** - shows the results of a reaction to identify the RS1183736 polymorphism in human genomic DNA carried out using primers without LNA-type modification in a real-time isothermal reaction with the addition of SybrGreen intercalating dye. Green indicates the system identifying the A allele, red the C allele.
**Fig.5****.** - shows the results of the reaction to identify the RS1183736 polymorphism in human genomic DNA carried out using primers with 1 nucleotide modification of the LNA type a real-time isothermal reaction with the addition of SybrGreen intercalating dye. Green indicates the system identifying the A allele, red the C allele.

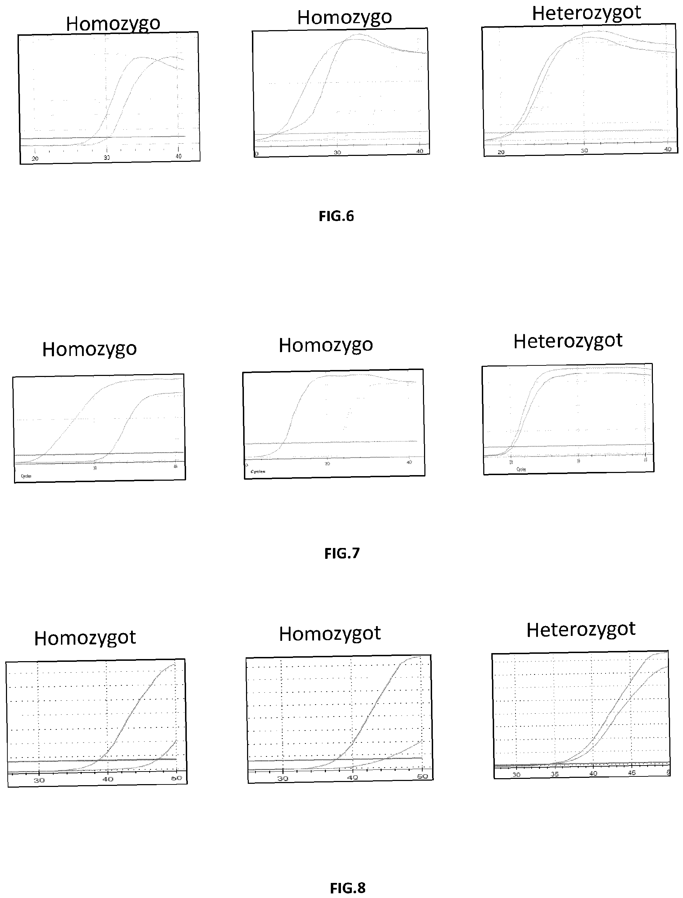

**Fig.6****.** - shows the results of the reaction to identify the RS1183736 polymorphism in human genomic DNA carried out using primers with LNA-type 3-nucleotide modification in a real-time isothermal reaction with the addition of SybrGreen intercalating dye. Green indicates the system identifying the A allele, red the C allele.
**Fig.7****.** - shows the results of the reaction to identify the RS1183736 polymorphism in human genomic DNA carried out using primers with 5 nucleotide modification of LNA type in real-time isothermal reaction using as a matrix isolated genomic DNA with a commercial kit. Green indicates the system identifying the A allele, red the C allele.
**Fig.8****.** - shows the results of a reaction to identify the RS1183736 polymorphism in human genomic DNA carried out using LNA-type 5-nucleotide modification primers in a real-time isothermal reaction using as a matrix a cheek swab sample directly added to the reaction. Green indicates the system identifying the A allele, red the C allele.

**SEQ. 1.** - shows the nucleotide sequence of the FIP primer.
**SEQ. 2.** - shows the nucleotide sequence of the BIP_Mut primer identifying the C allele, where the nucleotides preceded by "+" are LNA-type bases and the underlined nucleotide is a polymorphic C nucleotide.
**SEQ. 3.** - shows the nucleotide sequence of the BIP_WT primer identifying the A allele, where the nucleotides preceded by "+" are LNA-type bases and the underlined nucleotide is a polymorphic A nucleotide.
**SEQ. 4.** - shows the nucleotide sequence of the F3 primer.
**SEQ. 5.** - shows the nucleotide sequence of primer B3
**SEQ. 6.** - shows the nucleotide sequence of the LoopF primer.
**SEQ. 7.** - shows the nucleotide sequence of the LoopB primer.
**SEQ. 8.** - shows the nucleotide sequence of the human genomic DNA fragment flanking the RS1183736 polymorphism at position chr1:55717490 (NCBI), the letter N indicates the mutation site corresponding to nucleotide C or A
**SEQ. 9.** - shows the nucleotide sequence of the BIP_Mut primer identifying the C allele, where the underlined nucleotide is the polymorphic C nucleotide.
**SEQ. 10.** - shows the nucleotide sequence of the BIP_WT primer identifying the A allele, where the underlined nucleotide is the polymorphic nucleotide of A
**SEQ. 11.** - shows the nucleotide sequence of the BIP_Mut primer identifying the C allele, where the nucleotides preceded by "+" are LNA-type bases and the underlined nucleotide is a polymorphic C nucleotide.
**SEQ. 12.** - shows the nucleotide sequence of the BIP_WT primer identifying the A allele, where the nucleotides preceded by "+" are LNA-type bases and the underlined nucleotide is a polymorphic A nucleotide.
**SEQ. 13.** - shows the nucleotide sequence of the BIP_Mut primer identifying the C allele, where the nucleotides preceded by "+" are LNA-type bases and the underlined nucleotide is a polymorphic C nucleotide.
**SEQ. 14.** - shows the nucleotide sequence of the BIP_WT primer identifying the A allele, where the nucleotides preceded by "+" are LNA-type bases and the underlined nucleotide is a polymorphic A nucleotide.

The invention is illustrated by the following examples of use, which do not constitute a limitation of the invention

### Example 1

The object of the invention is primers used for the detection of SNP RS1183736 in human genomic DNA at position chr1:55717490 (NCBI). Seven DNA oligonucleotides have been designed for this purpose:
- An internal oligonucleotide called FIP that enables loop formation in the isothermal reaction,
- An internal oligonucleotide named BIP_Mut that enables loop formation in the isothermal reaction, additionally determining the identification of strands with C-type polymorphism
- An internal oligonucleotide named BlP_WT that enables loop formation in the isothermal reaction, additionally determining the identification of strands with A-type polymorphism
- external oligonucleotides named F3 and B3 to be used as primers that initiate the replacement of a duplex strand by a newly synthesized progeny strand in an isothermal reaction,
- Looping oligonucleotides named LoopF and LoopB to be used as complementary sequences to the loop-formed regions, increasing the speed of the isothermal reaction.

The sequences of the internal primers BIP_Mut and BIP_WT were enriched with modified LNA-type nucleotides, schematically designated in the sequence as +C+A+C+A+G or +C+A+A+A+G (SEQ2 and SEQ3). The modification involves "locking" the ribose ring in the aforementioned nucleotides by forming a methylene bridge between the 2'-O and 4'-C ribose atoms as shown in FIG1. The introduction of such a modification facilitated pairing with the complementary nucleotide strand, increased the stability of the resulting duplex in the amplification reaction, raised the temperature of primer attachment and consequently increased the specificity of the designed oligonucleotides.

External primer F3 (SEQ4) is a primer with a sequence starting from the 5' end relative to the leading strand of the target nucleic acid sequence (SEQ8). External primer B3 (SEQ5) is a primer with a sequence starting at the 3' end relative to the leading strand of the target nucleic acid sequence (SEQ8). The external primers F3 and B3 flank the regions amplified by the internal primers, are short and present in the reaction at a lower concentration, allowing them to bind to the matrix more slowly than the internal primers. This in turn leads to the initiation of the replacement of the duplex strand by the newly synthesized progeny strand. Their main function is to move the strand. The internal primers BIP_Mut and BIP_WT (SEQ2 and SEQ3) are primers that determine the specificity to attach to a strand with the C or A allele, respectively, within the RS1183736 polymorphism in human genomic DNA. The internal BIP (SEQ2 and SEQ3) and FIP (SEQ1) primers contain fragments in their sequence that are complementary to the sense and nonsense strands, enabling looping. LoopF and LoopB looping primers are complementary to regions with a formed loop. Their use significantly speeds up the reaction, as they bind to fragments of the target sequence not covered by internal primers, resulting in a faster overall amplification process
Appropriately designed and modified primers provide high specificity of the system against human genes other than the RS1183736 sequence at the chr1:55717490 position and genomic DNA of domestic and farm animals, as confirmed by the in silico analysis presented in FIG2.

Identification of SNP RS1183736 is carried out using two independent amplification reactions carried out in parallel at a single temperature. The first reaction using the FIP, BIP_Mut, F3, B3, LoopF and LoopB primer set (SEQ1, SEQ2, SEQ4, SEQ5, SEQ6, SEQ7) identifies the presence of the C allele, while the FIP, BIP_WT, F3, B3, LoopF and LoopB set (SEQ1, SEQ3, SEQ4, SEQ5, SEQ6, SEQ7) identifies the presence of the A allele. The results obtained after using both sets of primers to genotype human DNA for the RS1183736 polymorphism are shown in FIG3

In parallel, the identification reaction was carried out with primers with the same nucleotide sequence as above, but without LNA-type modifications (SEQ1, SEQ4, SEQ5, SEQ6, SEQ7, SEQ9, SEQ10, FIG4), with one LNA-type nucleotide in BIP_Mut and BIP_WT primers (SEQ1, SEQ4, SEQ5, SEQ6, SEQ7, SEQ11, SEQ12, FIG5) and three LNA-type nuclotides in BIP_Mut and BIP_WT primers (SEQ1, SEQ4, SEQ5, SEQ6, SEQ7, SEQ3, SEQ14, FIG6). A comparison of the results for the reaction with and without LNA-type modifications shows a definite improvement in the specificity of the system with LNA-type modification at five nucleotides in the BIP_Mut and BIP_WT primers relative to the other systems.

The use of primer sets, differing in the BIP_Mut or BIP_WT primer used, is shown in FIG3, FIG4, FIG5 and FIG6 by identifying homozygous genomic DNA for the presence of a C allele (C/C) or an A allele (A/A) in RS1183736 and heterozygous genomic DNA with one C allele and one A allele (C/A)

The identification analysis of the RS1183736 SNP polymorphism was carried out using a real-time isothermal DNA amplification reaction. Detection was made possible by using SybrGreen intercalating dye and observing the increase in fluorescence intensity as the reaction was carried out.

A detectable tracer or reporter molecule can also be attached to the BIP_Mut and BIP_WT primer for detection of the otzed products. Typical markers that can be used for probes include: radioactive isotopes, enzyme substrates, cofactors, ligands, chemiluminescent or fluorescent agents, haptens and enzymes. Identification can also be done using electrophoretic, densitometric or colorimetric methods.

### Example 2

In the example presented above of SNP identification using primers with type-5 modification of LNA molecules, the method of DNA amplification by real-time isothermal reaction with the addition of SybrGreen intercalating dye was used. The presented modification of type 5 LNA molecules in oligonucleotide can also be used in any method of amplification of any gene fragment with SNP polymorphism such as polymerase chain reaction (PCR), real-time PCR (qPCR), reverse transcriptase polymerase chain reaction (RT-PCR), real-time reverse transcriptase polymerase chain reaction (RT-qPCR), isothermal reaction (ex. LAMP), real-time isothermal reaction (e.g. qLAMP), isothermal reaction with reverse transcriptase (e.g. RT-LAMP), isothermal reaction with reverse transcriptase in real-time(e.g. RT-qLAMP), ligase chain reaction or transcription-mediated amplification (TMA)

The applied modification in the primers using the appropriate components in the reaction mixture allows the use for identification of biological samples both after isolation of genetic material with known commercial DNA extraction kits and directly from biological samples suspended in saline or other transport buffers with low salt and stabilizing substances. Samples can be analyzed directly from buccal swabs, saliva or other clinical materials. The absence of cross-reactions of selected primers with genomic DNA other than the intended molecular target allows with high sensitivity, specificity and specificity to identify SNPs in the sample. A comparison of results for isolated and direct samples - taken directly from the swab, using the identification of the RS1183736 polymorphism as an example, is presented in FIG7 and FIG8, respectively.

### Literature

1. G. Wang, E. Gunic, J.-L. Girardet, V. Stoisavljevic, Conformationally locked nucleosides. Synthesis and hybridization properties of oligodeoxynucletides containing 2P4P-C-bridged 2P-deoxynucleosides, Bioorg. Med. Chem. Lett. 9 (1999) 1147^1150.
2. Jensen et al. (2011) Evaluation of two commercial global miRNA expression profiling platforms for detection of less abundant miRNAs. BMC Genomics. 12:435. doi: 10.1186/1471-2164-12-435.
3. P. Gill, A. Ghaemi. Nucleic acid isothermal amplification Technologies - a review. Nucleos Nucleot Nucl Acids 2008; 27: 224-243
4. NCBI https://www.ncbi.nlm.nih.gov/snp/rs1183736

**ID SEQ1**
   ACACTATGCTACGGCAATATGCACGGAGAATCTATGAACACTC
**ID SEQ2**
   +C+A+C+A+GAACCATGCCCGAGAGAACCCACATAAGTCTTTCCAAGT
**ID SEQ3**
   +C+A+A+A+GAACCATGCCCGAGAGAACCCACATAAGTCTTTCCAAGT
**ID SEQ4**
   TGTCATTCCTGGCATGATG
**ID SEQ5**
   CACTGACAAGCCACCATT
**ID SEQ6**
   ACAGGACAGTCCTAGGCATT
**ID SEQ7**
   TCAAGGGCTCATGTTTCTCAA
**ID SEQ8** 
TCATGTATTTGGAATGAGGATATTAGCAGTATTTATTTCATGGTATGGTTCTCAGAACTCAGTGTGTTTATATACATG
AAGAGCTTAGGAAAGTATTTGGTTAGTCAGCACTATATAAGTTGTCATTCCTGGCATGATGTTTAGTAGTATTATCA
CTGTGTATAGTCATGACTTATCTTCAATTTTAAGCAATCTTTAATTGATACGGAGAATCTATGAACACTCCAAATGCC
TAGGACTGTCCTGTGCATATTGCCGTAGCATAGTGTGAAATTTCCTGAACTTTTCAGGCANAGAACCATGCCCGA
GAGAAATAGAAGAATAAAAAAGTACTCAAGGGCTCATGTTTCTCAAACTTGGAAAGACTTATGTGGGGGAGGAA
TGGTGGCTTGTCAGTGCTATATACAGAGTTAGAGAATAAAGACAGCATGTTTTTACAGAATATCACATTTACTAGG
AAAATGTAAGTAACATAACATTAGGTAATATTGAATCATCCAGAAATATCATAGAATAAAGGTAAAGTTGACATAAT
**ID SEQ9**
   CACAGAACCATGCCCGAGAGAACCCACATAAGTCTTTCCAAGT
**ID SEQ10**
   CAAAGAACCATGCCCGAGAGAACCCACATAAGTCTTTCCAAGT
**ID SEQ11**
   CA+CAGAACCATGCCCGAGAGAACCCACATAAGTCTTTCCAAGT
**ID SEQ12**
   CA+AAGAACCATGCCCGAGAGAACCCACATAAGTCTTTCCAAGT
**ID SEQ13**
   C+A+C+AGAACCATGCCCGAGAGAACCCACATAAGTCTTTCCAAGT
**ID SEQ14**
   C+A+A+AGAACCATGCCCGAGAGAACCCACATAAGTCTTTCCAAGT

## Claims

**1.** A method of modifying primers for isothermal amplification reaction using LNA-type nucleotides using the example of polymorphism RS1183736 in human genomic DNA, in chromosome 1 at position chr1:55717490 (NCBI), which includes:
- The nucleotide sequence of the FIP primer shown on SEQ1,
- The nucleotide sequence of the BIP_Mut primer identifying the C allele shown on SEQ2,
- The nucleotide sequence of the BIP_WT primer identifying the A allele shown on SEQ3,
- The nucleotide sequence of the F3 primer shown on SEQ4,
- The nucleotide sequence of the B3 primer shown on SEQ5,
- The nucleotide sequence of the LoopF primer shown on SEQ6,
- The nucleotide sequence of the LoopB primer shown on SEQ7.

**2.** The method according to claim 1, **characterized in that** the ribose ring in the 5 nucleotides of the primer is blocked: the nucleotide corresponding to the polymorphic allele/mutation and the four nucleotides in the immediate vicinity from the 5' and 3' sides of the oligonucleotide sequence, as shown in SEQ.1 and SEQ.2, by forming a methylene bridge between the 2'-O and 4'-C atoms.

**2.** The method according to claim 1, **characterized in that** the primers defined above are specific to a polymorphic sequence involving a single-nucleotide polymorphism with a C allele (mutant version) and an A allele (wild-type version).

**3.** The method of detecting and identifying a single-nucleotide mutation using RS1183736 as an example in human genomic DNA, including steps:
a) First reaction DNA amplification using primers with SEQ.1, SEQ.2, SEQ.4, SEQ.5, SEQ.6, SEQ.7.
b) The second reaction amplified DNA using primers with SEQ.1, SEQ.3, SEQ.4, SEQ.5, SEQ.6, SEQ.7.
c) Detection of the complexes formed by observing the increase in fluorescence of the intercalating dye, which is added to the reaction mixture.

**4.** The method according to claim 3, **characterized in that** the target gene material is swab, saliva, blood, tissue or other clinical samples containing genomic DNA.

**5.** A kit for use in the manner defined above, containing:
a) Starters
b) DNA polymerase with cofactor
c) Deoxyribonucleoside triphosphates
d) Reaction buffer
e) SybrGreen or EvaGreen intercalating dye.

**6.** The kit according to claim 5, **characterized in that it** contains reagents required for the amplification steps of hybridization and DNA strand synthesis.

**7.** Primers containing SEQ.1 - SEQ.7 for use in detecting and identifying a single-nucleotide mutation using RS1183736 as an example in human genomic DNA.
